(19) Europäisches Patentamt
European Patent Office
Office européen des brevets

(11) Veröffentlichungsnummer: **0 229 394**
**B1**

# (12) EUROPÄISCHE PATENTSCHRIFT

(45) Veröffentlichungstag der Patentschrift: **24.10.90**

(51) Int. Cl.[5]: **C07C 67/54**, C07C 69/734, C07C 67/60

(21) Anmeldenummer: **86118108.9**

(22) Anmeldetag: **29.12.86**

## (54) Reinigungsverfahren für einen p-Methoxyzimtsäureester.

(30) Priorität: **15.01.86 CH 128/86**
**16.10.86 CH 4137/86**

(43) Veröffentlichungstag der Anmeldung: **22.07.87 Patentblatt 87/30**

(45) Bekanntmachung des Hinweises auf die Patenterteilung: **24.10.90 Patentblatt 90/43**

(84) Benannte Vertragsstaaten: **AT BE CH DE ES FR GB IT LI NL SE**

(56) Entgegenhaltungen: **EP-A- 0 165 521**

(73) Patentinhaber: **L. GIVAUDAN & CIE Société Anonyme, CH-1214 Vernier-Genève(CH)**

(72) Erfinder: **Gonzenbach, Hans Ulrich, Dr., 61bis rue de Lyon, CH-1203 Genéve(CH)**
Erfinder: **Schudel, Peter, Dr., Gutshalde, CH-8624 Grüt(CH)**
Erfinder: **Schwarzenbach, Rolf, Köhlbergstrasse 1, CH-8405 Winterthur(CH)**

(74) Vertreter: **Urech, Peter, Dr. et al, Grenzacherstrasse 124 Postfach 3255, CH-4002 Basel(CH)**

EP 0 229 394 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Der 2-Aethylhexyl-p-methoxyzimtsäureester, also die Verbindung der Formel

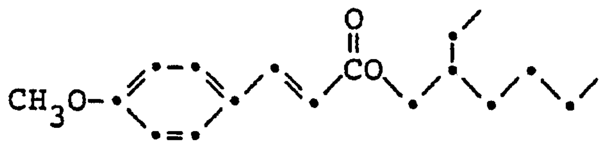

I

und zwar dessen E- und Z-Isomeres und beliebige Gemische dieser Isomeren stellen bekannte Lichtschutzmittel für den UV-B-Bereich dar.

Die Herstellung der Verbindung I kann z.B. erfolgen gemäss:

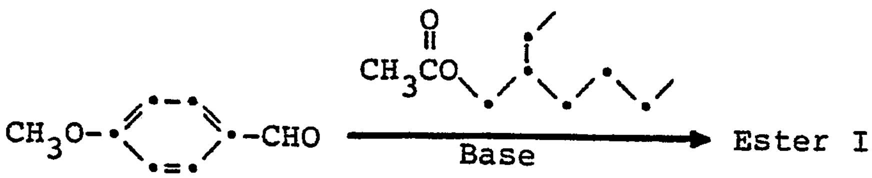


oder gemäss:

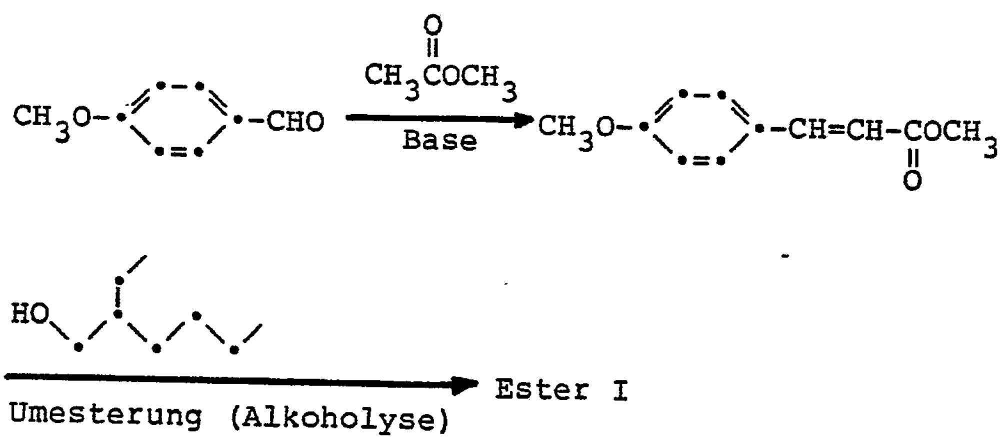


In jedem Fall wird das Rohmaterial nach der Herstellung zweckmässigerweise gereinigt, und zwar insbesondere durch Destillation, wobei z.B. nicht umgesetztes oder im Ueberschuss vorhandenes Ausgangsmaterial abgetrennt wird. Ein weiterer Zweck dieser Reinigung liegt darin, olfaktisch einwandfreies Material zu erhalten. Der Ester I wird ja vorwiegend in kosmetischen Präparaten und Sonnenschutzmitteln für die menschliche Haut verwendet und sollte deshalb geruchlich möglichst neutral sein.

In letzter Zeit sind Stimmen laut geworden, die diesem Ester - je nach Provenienz - mutagene Eigenschaften zuschreiben, siehe z.B. A.M. Bonin et al., Mutation Research 105 (1982), 303-308.

Diese mutagenen Eigenschaften wurden dabei aus den Ergebnissen des bekannten Ames-Tests gefolgert, siehe z.B. A.M. Bonin et al., loc. cit. und B.N. Ames et al., Mutation Research 31 (1975), 347 - 364.

Dieser Test stellt bekanntlich ein von Ames et al. entwickeltes Verfahren zur Bestimmung der Mutagenität chemischer Verbindungen dar. Untersuchungsobjekt ist eine Bakterien-Defektmutante, die sich unter dem Einfluss von Mutagenen zur normalen Mutante (Wildtyp) zurückverwandeln kann.

Es wird von A.M. Bonin et al., loc. cit. vermutet, dass im Falle des Esters I eine manchmal in diesem Ester I vorhandene mutagene Spurenkomponente für das Eintreten einer Ames-positiven Reaktion verantwortlich ist; deren Natur ist aber unbekannt.

Es wurde nun gefunden, dass diese Spurenkomponente aus dem Ester I immer verschwindet, entfernt bzw. "unschädlich" gemacht wird, wenn man den Ester I zusammen mit einem Phenol erhitzt, oder - vorzugsweise - destilliert. Am so "gereinigten" Ester I vorgenommene Ames-Tests zeitigen in der Folge in allen Fällen negative Resultate.

Die Erfindung betrifft demgemäss ein Verfahren zur Reinigung des 2-Aethylhexyl-p-methoxyzimtsäureesters, das dadurch gekennzeichnet ist, dass man den Ester in Anwesenheit eines Phenols erhitzt, vorzugsweise destilliert.

Die Destillation ist zweckmässigerweise zugleich die Reinigungsstufe des Rohproduktes der Synthese, doch kann natürlich auch (anderes) Material irgendwelcher Provenienz einer Destillation unterworfen werden.

Als Phenol wird beispielsweise ein (sterisch) gehindertes Phenol ("hindered phenol"), insbesondere ein mindestens mono-, insbesondere o- oder p-substituiertes Phenol, insbesondere ein mindestens monosubstituiertes Phenol der Formel

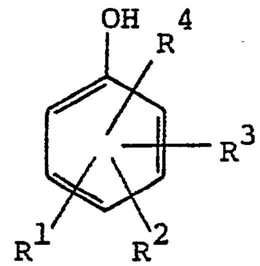

II

worin $R^1$, $R^2$ und $R^3$ gleich oder verschieden sind und Wasserstoff, $C_1$-$C_{12}$-Alkyl, $C_2$-$C_{12}$-Alkenyl, $C_{1-6}$-Alkoxy, Hydroxy oder Aryl, z.B. Phenyl oder Naphthyl, bedeuten, und $R^4$ Wasserstoff oder den Rest $(CH_2)_n$-COOR mit R=Wasserstoff oder $C_{1-18}$-Alkyl und n=0,1,2,3,4 oder den Rest $-(CH{=}CH)_n$-COOR mit R=Wasserstoff oder $C_{1-18}$-Alkyl und n=0,1,2 darstellt, benützt.

Die Reste $R^1$, $R^2$ und $R^3$ können in o-, m-, oder p-Stellung stehen. Bevorzugt ist die o- und die p-Stellung. Die Alkyl-, Alkenyl- und Alkoxyreste können linear oder, bevorzugt, verzweigt sein.

Zunächst werden unter dem Begriff "gehindertes Phenol" insbesondere Alkylphenole verstanden, bei denen am aromatischen Ring eines oder mehrere der H-Atome - vorzugsweise eine oder beide ortho-Stellungen bzw. die p-Stellung - durch gleiche oder verschiedene Alkylgruppen ersetzt sind. Beispiele von Alkylphenolen sind Kresole (o-, m-, p-) und Xylenole (2,3-; 2,4-; 2,5-; 2,6-; 3,4-; 3,5-Dimethylphenol), 4-tert.-Butylphenol, 2,6-di-tert. Butylphenol und Abkömmlinge davon, 2,6-Di-Tert.-butyl-p-kresol, Thymol (2-Isopropyl-5-methylphenol) und Carvacrol (2-p-Cymenol), 2-Isopropenyl-5-methylphenol sowie 4-tert.-Octylphenol, die Nonylphenole und die Dodecylphenole, ferner 2,6-di-tert.-Butyl-4-methylphenol (BHT), 2(und 3)-tert.Butyl-4-methoxyphenol (BHA), tert.Butyl-hydrochinon (TBHQ);

Als gehinderte Phenole kommen definitionsgemäss aber auch in Frage:

Gallussäure und deren Derivate (z.B. Ester wie (nieder-)Alkylester, Aether wie (nieder-)Alkyläther), polycyclische Phenole, z.B. 2- oder 3-cyclische, mit dem Strukturprinzip der Formel II, z.B. Tocopherole, oder komplexere Moleküle mit dem Strukturprinzip der Formel II, z.B. die vielen bekannten, o-(bevorzugt), m- oder p- (bevorzugt) Abkömmlinge des 2,6-di-t-Butylphenols, wie z.B.

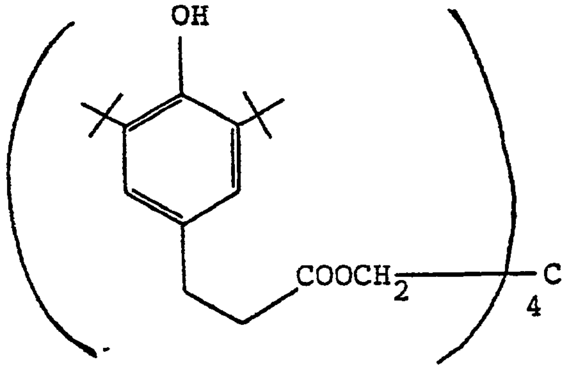

oder

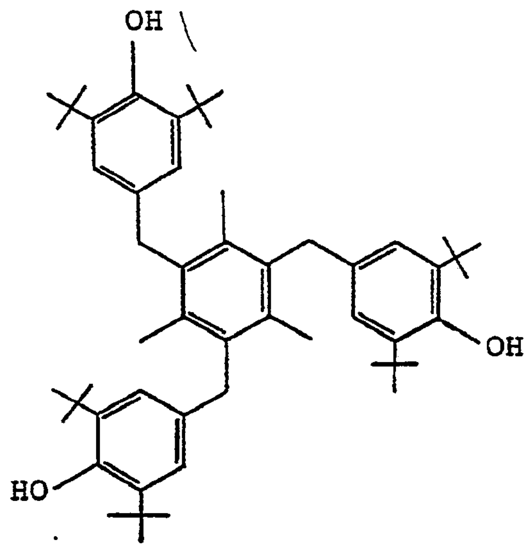

etc.

Geeignete gehinderte Phenole sind aber definitionsgemäss auch o- (bevorzugt), m- oder p- (bevorzugt) Hydroxyzimtsäuren oder o- (bevorzugt), m- oder p- (bevorzugt) Hydroxyzimtsäureester, insbesondere o- (bevorzugt), m- oder p- (bevorzugt) Hydroxyzimtsäure-$C_{1\text{-}18}$-alkyl-, phenyl-, benzyl- oder cyclohexylester, wobei der Phenylrest gegebenenfalls mit einem Alkylrest, z.B. einem $C_{1\text{-}4}$-Alkylrest, z.B. Methyl, Aethyl, Propyl oder Butyl o-, m- oder p-substituiert sein kann, und der Alkylester insbesondere einen $C_{1\text{-}18}$-Alkylester darstellt.

Bezüglich der Alkylreste gilt im übrigen das oben Gesagte.

Besonders gut geeignete gehinderte Phenole sind:
p-tert.Butyl-hydroxy-toluol (BHT), p-tert.Butyl-hydroxy--anisol (BHA), tert. Butyl-hydrochinon (TBHQ).

Die Art der Destillation ist nicht kritisch.

Obschon natürlich sogar einfache Destillationen (Gerade-aus-, Gleichstrom-, absteigende Destillation), z.B. für den Labormasstab, eingesetzt werden können, ist insbesondere für den industriellen Masstab Kolonnendestillation (Rektifikation) selbstverständlich vorzuziehen. Hierbei kommen die üblicherweise dazu verwendeten Kolonnen zum Einsatz, also beispielsweise
Bodenkolonnen, z.B. Siebbodenkolonnen,
Sprühkolonnen,
Drehbandkolonnen,
Spraypak-Kolonnen,
Spaltrohrkolonnen,
Füllkörperkolonnen.

Das Konstruktionsmaterial dieser Kolonnen ist nicht kritisch und ist zweckmässigerweise Glas, Quarzglas, Edelstahl, Keramik, polymerer Kunststoff, z.B. fluorierter Kunststoff.

Vorzugsweise werden Kolonnen mit Kolonnenfüllungen aus Edelstahl, z.B. vom Typ Meshpack (also geometrisch geordneter Packung) verwendet.

Man kann selbstverständlich sowohl stetig (kontinuierlich), aber auch unstetig (diskontinuierlich) rektifizieren.

Die Rektifikation erfolgt vorzugsweise unter vermindertem Druck, z.B. einem Druck von ca. 0,1 bis 5 mbar, insbesondere ca. 0,1 bis 2 mbar.

Man destilliert zweckmässigerweise in einem Temperaturbereich von ca. 150°C bis 230° C, insbesondere von ca. 170°C bis 200°C, denn der Ester I weist folgende physikalische Daten auf:
Siedepunkt
185 - 195 °C / 1 mbar
140 - 150 °C /0,1 mbar.

Die Destillationsgeschwindigkeit ist nicht kritisch, sie richtet sich insbesondere nach Grösse und Art der Installation; sie ist zu Beginn der Destillation üblicherweise langsamer und kann nach Abtrennen des Vorlaufs in üblicher Weise gesteigert werden [s.a. Römpps Chemielexikon, 8. Auflage, Franckh'sche Verlagshandlung, Stuttgart (1981), Band 2, Destillation, Kolonnen].

Die Menge des bei der Destillation zugesetzten gehinderten Phenols ist nicht kritisch. Sie beträgt aber zweckmässigerweise bloss ca. 0,01 bis 2 Gew.%, insbesondere ca. 0,05 bis 0,5 Gew.% des Esters I.

Zweckmässigerweise wird das Phenol in Form einer konzentrierten Lösung, z.B. einer 10 bis 40%-igen Lösung in einem geeigneten Lösungsmittel, zweckmässigerweise einem polaren hoch siedenden Lösungsmittel oder - vorzugsweise - gelöst in dem Ester I selber dem Destillationsgut zugegeben. Die Zugabe eines flüssigen Phenols kann aber auch in unverdünnter Form, z.B. durch Einspritzen erfolgen. Die kontinuierliche Zugabe ist bevorzugt.

Im Falle des blossen Erhitzens des Esters I sind die zweckmässigen Parameter:
Menge Phenol: 0,05 - 2 Gew.%, insbesondere 0,1 - 1 Gew.%
Temperatur: 150 - 230°C, vorzugsweise 170-200°C
Zeit: mehrere Stunden, z.B. 3-30, insbesondere 6-20 Stunden *
Druck: Atmosphärendruck, Ueberdruck oder verminderter Druck
Gefäss: nicht-kritisch
Atmosphäre: nicht kritisch; Inertatmosphäre ist aber bevorzugt.

Ames-Test

Mikroorganismus

histidin-benötigender Stamm ("bacterial tester strain"): Salmonella typhimurium TA 1538

Kulturen:

a) tiefgefrorene Stammkultur ("frozen permanents") in Nährlösung NB (s.u.), 8% DMSO, Aufbewahrung bei -80°C

*Für die diskontinuierliche Destillation sind diese angegebenen Zeitspannen ebenfalls zweckmässig. Für kontinuierliches Arbeiten kann die Verweilzeit aber auch viel kürzer sein. In diesem Fall können oft schon wenige Minuten, z.B. 2-10 Minuten genügen. Wie oben gesagt, ist die Destillationsgeschwindigkeit nicht kritisch, und deshalb auch die Zeit nicht, da diese im wesentlichen von der Geschwindigkeit abhängt.

b) Schrägagar-Subkulturen mit Nährgar NBA nach Inkubation von 24 Stunden bei 37°C mit üblichem Spontanfrequenzbereich von bis zu 30 Kolonien pro Platte (siehe Maron and Ames, Mutation Res. 113 (1983), 173 - 215).

Aufbewahrung bei 4°C, periodische diesbezügliche Prüfung auf Spontanfrequenzbereich, UV-Sensitivität und Kristallviolett-Sensitivität gemäss Maron und Ames, loc cit., Seiten 179/180.

c) Versuchskulturen: Wachstum über Nacht in Nährlösung NB bei 37°C im Schüttelwasserbad. Zelldichte 1-2 x $10^9$ Zellen x $ml^{-1}$, bestimmt durch Ausplattung einer geeigneten Verdünnung auf Komplettmedium NBA.

Medien

a) Nährmedium NB: 25 g/l Nährlösung Oxoid Nr. 2 [Oxoid Ltd. Basingstoke, Hants, GB] (Komplettmedium),

b) Nähragar NBA : NB + 15 g/l Difco Bacto Agar [Difco Laboratories Detroit, Michigan],

c) Vogel-Bonner Minimal-Agarplatten [Gibco Ltd., P.O.Box 35, Paisley, Schottland] (histidinfrei)
1 l enthalten:
- 20 ml folgender Salzlösung:

| | | |
|---|---|---|
| 10 g $MgSO_4 \times 7H_2O$<br>109 g Zitronensäure x $1H_2O$<br>500 g $K_2HPO_4$<br><br>175 g $NaNH_4HPO_4 \times 4H_2O$<br>670 ml $H_2O$ | } | im Autoklaven 20 Minuten auf 120°C erhitzt |

- Glucose : 20 g/l steril filtriert
- Difco Bacto Agar: 15 g/l

d) Weichagar ("Top Agar"): NaCl 5,0 g/l
Difco Bacto Agar 7,0 g/l
0,05 mM Biotin
0,05 mM L-Histidin (als "Starter"

Versuchsdurchführung

Die zu untersuchenden Proben des Esters I werden mit Dimethylsulfoxid in Glasröhrchen so verdünnt, dass folgende Konzentrationsreihe entsteht: 750µl [I]/ml, 500µl [I]/ml, 300µl [I]/ml, 100µl [I]/ml und 30µl [I]/ml.

Es werden je 0,1 ml davon zu 2,0 ml flüssigem (Wasserbadtemperatur 45±1°C) Weichagar pipettiert, so dass pro Gläschen der Ester I in Mengen von 75µl, 50µl, 30µl, 10µl und 3µl vorhanden ist.

0,1 ml der Kultur c) werden in diese Gläschen gegeben und die Gläschen jeweils sofort auf Petrischalen vom Durchmesser 8,5 cm (enthaltend 20 ml Vogel-Bonner Minimal-Agar) gegeben. Pro einzelne Verdünnung werden 4 Petrischalen ausgeplattet. Nach Verfestigung der oberen Agarschicht werden die Schalen umgekehrt und während 2 Tage bei 37°C inkubiert. 2 Tage nach der Ausplattung werden die sichtbaren Kolonien mit blossem Auge ausgezählt.

Als Kontrollen dienen Schalen, bei deren Herstellung anstelle der Verdünnungsreihen nur je 0,1 ml DMSO und 0,1 ml TA 1538 eingesetzt wurden.

Es werden definiert:

$$\text{MF (Mutationsfrequenz)} \longrightarrow \frac{\text{Anzahl Kolonien in den Proben [I]}}{\text{Anzahl Kolonien in der Kontrolle}}$$

"Ames-positiv" mindestens Verdopplung der Anzahl Kolonien, wenn verglichen mit der Kontrolle
"Ames-negativ" die Testplatte enthält weniger als die doppelte Menge der Kolonien der Kontrolle.

Beispiel 1

5 kg Ester I werden in einen 10 Liter 4-Halskolben gegeben, welcher mit Kapillare, Thermometer, Tropftrichter und Destillationskolonne versehen ist. Die Kolonne weist eine Höhe von 2.30 m und einen Durchmesser von 70 mm auf und ist mit Drahtgeflecht vom Typ SULZER BX Inox gefüllt. Am Kolonnenkopf kann das Rücklaufverhältnis von 1:5 am Anfang der Destillation bis zu 1:1 gegen Ende der Destillation geregelt werden. Als gehindertes Phenol werden 75 g einer 33%-igen Lösung von Butylhydroxytoluol (BHT) bzw. Butylhydroxyanisol (BHA) kontinuierlich über die ganze Destillationsdauer von 6 Stunden zugegeben. Der Druck beträgt am Kolonnenkopf ca. 1 mbar, die Temperatur daselbst 185 - 195°C und in der Blase 216°C. Es wird zu Fraktionen von ca. 500 g fraktioniert, welche Fraktionen einer gaschromatographischen Analyse unterzogen werden und überdies geruchlich beurteilt werden. Die organoleptisch akzeptablen Fraktionen weisen gemäss gaschromatographischer Analyse einen Gehalt von mehr als 98% Ester I auf und werden am Schluss vereinigt.

Ames-Test

Ergebnisse

| Versuch Nr. | Zusatz | µl [I]/Platte | MF-Werte | | | | |
|---|---|---|---|---|---|---|---|
| | | | 3 | 10 | 30 | 50 | 75 |
| 1 | - | Ester I* | 3,0 | 6,8 | 15,2 | 13,0 | 12,0 |
| 2 | BHT | Ester I | 0,8 | 0,6 | 0,6 | 0,8 | 1,0 |
| 3 | BHA | Ester I | | 1,0 | 1,0 | 1,8 | |

* Ausgangsmaterial für die Versuche 2 und 3

Beispiel 2

Wird der Ester I*, in welchem 0,5 Gew.% BHT homogen gelöst sind, in einem offenen Gefäss erhitzt, werden analoge Resultate wie beim Destillieren erhalten.

Ames Test

Ergebnisse

| Versuch Nr. | Zusatz | µl [I]/Platte | MF-Werte | | | | |
|---|---|---|---|---|---|---|---|
| | | | 3 | 10 | 30 | 50 | 75 |
| 1 | BHT | Ester I* | 3,0 | 6,8 | 15,2 | 13,0 | 12,0 |
| 4 | BHT | Ester I 150°C/8h | 1,4 | 2,2 | 3,2 | 3,4 | 2,6 |
| 5 | BHT | Ester I 150°C/16h | 1,0 | 1,6 | 3,0 | 2,8 | 1,6 |
| 6 | BHT | Ester I 180°C/8h | 0,8 | 0,8 | 1,4 | 1,0 | 1,0 |

* Ausgangsmaterial für die Versuche 4, 5 und 6

Beispiel 3

In folgender Versuchsreihe wurden jeweils 75g Ester I** mit 0,5 Gew.% eines Phenols versetzt und in einem mit Siedekapillare und Destillationsbrücke versehenen Kolben destilliert. Die Siedetemperature betrug 160±5°C, die Badtemperatur 195-215°C und das Vakuum 0,2-0,25 mmHg. Die Ausbeuten waren quantitativ; die Destillate wurden einem Ames Test unterworfen.

Ames Test

Ergebnisse

| Versuch Nr. | Zusatz | µl [I]/Platte | MF-Werte 3 | 10 | 30 | 50 | 75 |
|---|---|---|---|---|---|---|---|
| 7 | - | Ester I** | 4,3 | 12,2 | 27,3 | 30,2 | 20,8 |
| 8 | Hydrochinon | Ester I | 1,2 | 0,8 | 2,0 | 2,0 | 1,2 |
| 9 | TBHQ | Ester I | 0,8 | 1,8 | 2,3 | 1,7 | 1,5 |
| 10 | p-Hydroxy-zimtsäure-2-äthylhexyl-ester | Ester I | 1,3 | 1,7 | 2,5 | 2,3 | 2,5 |
| 11 | Gallussäure-propylester | Ester I | 0,7 | 0,5 | 0,2 | | |
| 12 | Gallussäure-octylester | Ester I | 0,8 | 1,0 | 1,2 | 0,8 | 1,0 |
| 13 | Gallussäure-dodecylester | Ester I | 1,0 | 1,7 | 2,0 | 2,3 | 2,0 |

| | | | | | | | |
|---|---|---|---|---|---|---|---|
| 14 | p-Hydroxy-m,m-di-tert.butyl-phenyl-propionsäure-stearylester | Ester I | 1,3 | 2,0 | 2,5 | 2,3 | 1,7 |
| 15 | p-Hydroxy-zimtsäure-n-butylester | Ester I | 2,3 | 3,0 | 5,0 | 2,7 | 2,7 |
| 16 | p-Hydroxy-zimtsäure-methylester | Ester I | 2,3 | 3,7 | 4,7 | 2,3 | 2,7 |

** Ausgangsmaterial für die Versuche 8 - 16

## Patentansprüche

1. Verfahren zur Reinigung von 2-Aethylhexyl-p-methoxyzimtsäureester, dadurch gekennzeichnet, dass man den Ester in Anwesenheit eines Phenols erhitzt, vorzugsweise destilliert.

2. Verfahren nach Anspruch 1, dadurch gekennzeichnet, dass man den Ester in Anwesenheit eines gehinderten Phenols erhitzt, vorzugsweise destilliert.

3. Verfahren nach Anspruch 1 oder 2, dadurch gekennzeichnet, dass man ein mindestens (o- oder p-)monosubstituiertes Phenol benützt.

4. Verfahren nach Anspruch 1, 2 oder 3, dadurch gekennzeichnet, dass man als gehindertes Phenol eine mindestens monosubstituierte phenolische Verbindung der allgemeinen Formel

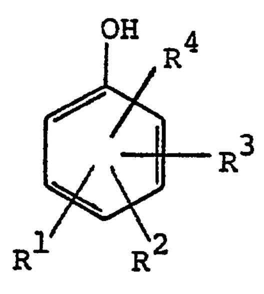

II

worin $R^1$, $R^2$ und $R^3$ gleich oder verschieden sind und Wasserstoff, $C_1$-$C_{12}$-Alkyl, $C_2$-$C_{12}$--Alkenyl, $C_{1-6}$-Alkoxy, Hydroxy oder Aryl, vorzugsweise Phenyl oder Naphthyl, bedeuten, und $R^4$ Wasserstoff oder den Rest $(CH_2)_n$-COOR mit R=Wasserstoff oder $C_{1-18}$-Alkyl und n=0,1,2,3,4 oder den Rest $-(CH=CH)_n$-COOR mit R=Wasserstoff oder $C_{1-18}$-Alkyl und n=0,1,2 darstellt,
benützt.

5. Verfahren nach einem der Ansprüche 1 bis 4, dadurch gekennzeichnet, dass man als gehindertes Phenol ein Alkylphenol, insbesondere 2,6-di-tert.Butyl-4-methyl-phenol (BHT), 2(und 3)tert. Butyl-4-methoxy-phenol (BHA) oder tert.Butyl-hydrochinon (TBHQ) verwendet.

6. Verfahren nach einem der Ansprüche 1 bis 4, dadurch gekennzeichnet, dass man als gehindertes Phenol eine Hydroxyzimtsäure oder einen Hydroxyzimtsäureester benützt.

7. Verfahren nach einem der Ansprüche 1 bis 4, dadurch gekennzeichnet, dass man als gehindertes Phenol Gallussäure oder ein Derivat davon benutzt.

8. Verfahren nach einem der Ansprüche 1 bis 7, dadurch gekennzeichnet, dass man unter vermindertem Druck, zweckmässigerweise bei ca. 0,1 bis 5 mbar, insbesondere bei ca. 0,1 bis 2 mbar destilliert.

9. Verfahren nach einem der Ansprüche 1 bis 8, dadurch gekennzeichnet, dass man das Phenol in einer Menge von 0,01 bis 2%, insbesondere von 0,05 bis 0,5% zusetzt.

10. Verfahren nach einem der Ansprüche 1 bis 9, dadurch gekennzeichnet, dass man in einer Kolonne destilliert.

## Claims

1. A process for the purification of 2-ethylhexyl p-methoxycinnamic acid ester, characterized by heating, preferably distilling the ester in the presence of a phenol.

2. A process according to claim 1, characterized in that the ester is heated, preferably distilled, in the presence of a hindered phenol.

3. A process according to claim 1 or 2, characterized in that an at least (o- or p-)monosubstituted phenol is used.

4. A process according to claim 1, 2 or 3, characterized in that an at least monosubstituted phenolic compound of the general formula

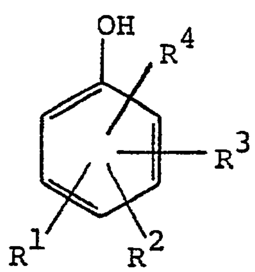

II

wherein $R^1$, $R^2$ and $R^3$ are the same or different and signify hydrogen, $C_1$–$C_{12}$-alkyl, $C_2$–$C_{12}$-alkenyl, $C_{1-6}$-alkoxy, hydroxy or aryl, preferably phenyl or naphthyl, and $R^4$ represents hydrogen or the residue $(CH_2)_n$–COOR with R = hydrogen or $C_{1-18}$-alkyl and n = 0, 1, 2, 3, 4 or the residue $-(CH{=}CH)_n$–COOR with R = hydrogen or $C_{1-18}$-alkyl and n = 0, 1, 2,
is used as the hindered phenol.

5. A process according to any one of claims 1 to 4, characterized in that an alkylphenol, especially 2,6-di-tert.butyl-4-methyl-phenol (BHT), 2(and 3)tert.butyl-4-methoxy-phenol (BHA) or tert.butyl-hydroquinone (TBHQ), is used as the hindered phenol.

6. A process according to any one of claims 1 to 4, characterized in that a hydroxycinnamic acid or a hydroxycinnamic acid ester is used as the hindered phenol.

7. A process according to any one of claims 1 to 4, characterized in that gallic acid or a derivative thereof is used as the hindered phenol.

8. A process according to any one of claims 1 to 7, characterized in that the distillation is carried out under reduced pressure, conveniently at about 0.1 to 5 mbar, especially at about 0.1 to 2 mbar.

9. A process according to any one of claims 1 to 8, characterized in that the phenol is added in an amount of 0.01 to 2%, especially of 0.05 to 0.5%.

10. A process according to any one of claims 1 to 9, characterized in that the distillation is carried out in a column.

## Revendications

1. Procédé pour purifier le p-méthoxycinnamate de 2-éthylhexyle, caractérisé en ce que l'on chauffe cet ester, et de préférence on le distille, en présence d'un phénol.

2. Procédé selon la revendication 1, caractérisé en ce que l'on chauffe l'ester, et de préférence on le distille, en présence d'un phénol qui est l'objet d'un empêchement stérique.

3. Procédé selon la revendication 1 ou 2, caractérisé en ce que l'on utilise un phénol au moins monosubstitué (en o ou en p).

4. Procédé selon la revendication 1, 2 ou 3, caractérisé en ce que l'on utilise en tant que phénol faisant l'objet d'un empêchement stérique un composé phénolique au moins monosubstitué de formule générale

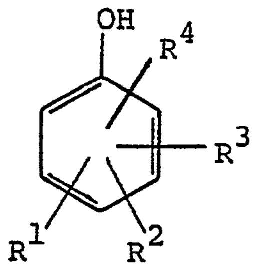

II

dans laquelle $R^1$, $R^2$ et $R^3$, ayant des significations identiques ou différentes, représentent chacun l'hydrogène, un groupe alkyle en $C_1$–$C_{12}$, alcényle en $C_2$–$C_{12}$ alcoxy en $C_1$–$C_6$, hydroxy ou aryle, de préférence phényle ou naphtyle, et $R^4$ représente l'hydrogène ou le groupe $(CH_2)_n$–COOR dans lequel R = hydrogène ou alkyle en $C_1$–$C_{18}$ et n = 0, 1, 2, 3, 4 ou le groupe –(CH=CH)$_n$–COOR dans lequel R = hydrogène ou alkyle en $C_1$–$C_{18}$ et n = 0, 1, 2.

5. Procédé selon l'une des revendications 1 à 4, caractérisé en ce que l'on utilise en tant que phénol objet d'un empêchement stérique un alkylphénol, en particulier le 2,6-di-tert-butyl-4-méthylphénol (BHT), le 2-(et 3)-tert-butyl-4-méthoxyphénol (BHA) ou la tert-butylhydroquinone (TBHQ).

6. Procédé selon l'une des revendications 1 à 4, caractérisé en ce que l'on utilise en tant que phénol objet d'un empêchement stérique un acide hydrocinnamique ou un ester hydrocinnamique.

7. Procédé selon l'une des revendications 1 à 4, caractérisé en ce que l'on utilise en tant que phénol objet d'un empêchement stérique l'acide gallique ou l'un de ses dérivés.

8. Procédé selon l'une des revendications 1 à 7, caractérisé en ce que l'on distille sous vide, de préférence sous un vide d'environ 0,1 à 5 mbar et plus spécialement d'environ 0,1 à 2 mbar.

9. Procédé selon l'une des revendications 1 à 8, caractérisé en ce que l'on ajoute le phénol en quantité de 0,01 à 2%, plus spécialement de 0,05 à 0,5%.

10. Procédé selon l'une des revendications 1 à 9, caractérisé en ce que l'on distille dans une colonne.